(19) Europäisches Patentamt
European Patent Office
Office européen des brevets

(11) Numéro de publication : **0 303 550 B1**

# (12) FASCICULE DE BREVET EUROPEEN

(45) Date de publication du fascicule du brevet : **22.04.92 Bulletin 92/17**

(51) Int. Cl.$^5$ : **C07C 209/48,** C07C 211/09

(21) Numéro de dépôt : **88420272.2**

(22) Date de dépôt : **29.07.88**

## (54) Procédé de fabrication de la méthyl-2 pentanediamine par hydrogénation du méthyl-2 glutaronitrile.

(30) Priorité : **12.08.87 FR 8711623**

(43) Date de publication de la demande : **15.02.89 Bulletin 89/07**

(45) Mention de la délivrance du brevet : **22.04.92 Bulletin 92/17**

(84) Etats contractants désignés : **AT BE CH DE ES FR GB GR IT LI LU NL SE**

(56) Documents cités :
**CH-A- 245 678**
**FR-A- 1 445 034**
**US-A- 3 953 511**

(73) Titulaire : **RHONE-POULENC CHIMIE**
**25, quai Paul Doumer**
**F-92408 Courbevoie Cédex (FR)**

(72) Inventeur : **Cordier, Georges**
**50, Chemin des Hermières**
**F-69340 Francheville (FR)**

(74) Mandataire : **Varnière-Grange, Monique et al**
**RHONE-POULENC INTERSERVICES Service Brevets Chimie Centre de Recherches des Carrières B.P. 62**
**F-69192 Saint-Fons Cédex (FR)**

EP 0 303 550 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de fabrication de la méthyl-2 pentanediamine par hydrogénation du méthyl-2 glutaronitrile.

Lors de la fabrication industrielle de l'adiponitrile par hydrocyanation du pentène-3 nitrile sont produites des quantités variables mais relativement importantes de méthyl-2 pentanediamine. Ce sous-produit peut être détruit, ce qui, bien évidemment, n'est pas satisfaisant sur le plan économique. Il peut également être valorisé, non pas en tant que tel, mais après transformation en produit utile. Parmi les valorisations qui paraissent les plus prometteuses, on peut citer la transformation du méthyl-2 glutaronitrile en isocyanate correspondant (méthyl-2 pentanediisocyanate) et, le cas échéant, en ses dérivés tels le biuret et le triisocyanate ; cette transformation passe par deux opérations successives, à savoir : l'hydrogénation du méthyl-2 glutaronitrile en méthyl-2 pentanediamine suivie de la phosgénation de la méthyl-2 pentanediamine en diisocyanate, selon les techniques décrites, par exemple, dans le brevet américain n° 3613 198. Une autre valorisation possible du méthyl-2 glutaronitrile réside dans sa transformation en méthyl-2 pentanediamine, qui peut être utilisée dans la fabrication de polyamides spéciaux.

Dans les cas envisagés, il est important d'obtenir lors de l'hydrogénation une sélectivité et une productivité élevées pour assurer la meilleure valorisation possible du méthyl-2 glutaronitrile.

La présente invention a pour but de proposer un procédé amélioré d'hydrogénation du méthyl-2 glutaronitrile en vue d'obtenir sélectivement la méthyl-2 pentanediamine.

L'hydrogénation des dinitriles aliphatiques saturés, le cas échéant alkyl- substitués est un réaction qui a donné lieu à de très nombreuses études antérieures. Ainsi, par exemple, dans la demande de brevet allemand n° 1 543 793, on a hydrogéné à 100°C et sous 300 atmosphères du méthyl-2 glutaronitrile en présence d'un catalyseur à base de cobalt de Raney, dans l'alcool absolu, en présence d'ammoniac anhydre pour obtenir de la méthyl-2 pentanediamine en mélange avec de la méthyl-3 pipéridine et des polymères de nature non identifiée avec les rendements respectifs suivants 80 : 18 : 2 (en pour cent), la durée de l'hydrogénation n'étant pas mentionnée.

Dans la demande de brevet français n° 2 306 202 on a hydrogéné à 110°C et sous 400 atmosphères du méthyl-2 glutaronitrile en présence de cobalt dans l'ammoniac liquide en alimentant le méthyl-2 glutaronitrile en continu, en faisant passer de l'hydrogène dans le cycle et en retirant le mélange réactionnel en continu ; la composition moyenne dudit mélange est la suivante : 90,9% de méthyl-2 pentanediamine, 8,2 % de méthyl-3 pipéridine et de l'ordre de 2 % de produits lourds (Cf. l'exemple 1 dit "exemple comparatif" de ladite demande). L'exemple 2 de cette même demande montre que toutes conditions égales par ailleurs, l'hydrogénation du méthyl-2 glutaronitrile conduite en présence d'un catalyseur à base de nickel ne permet pas d'obtenir sélectivement le méthyl-2 pentanediamine, puisqu'au contraire le mélange réactionnel ainsi obtenu renferme 90,1 % de méthyl-3 pipéridine contre 3,9 % de méthyl-2 pentanediamine et de 6 à 8 % de lourds. L'exemple 6 de cette même demande montre, en outre, qu'en opérant en discontinu, en l'absence d'ammoniac liquide, à 140°C sous 300 atmosphères, l'hydrogénation du méthyl-2 glutaronitrile en présence de nickel dans un milieu éthanolique renfermant de la soude, on obtient après 2 heures, de la méthyl-2 pentanediamine en mélange notamment avec de la méthyl-3 pipéridine, avec une sélectivité de 29,4 % (molaire) seulement.

En outre, dans le brevet américain n° 395 3 511, il est décrit un procédé multistade dans lequel l'hydrogénation du méthyl-2 glutaronitrile, qui constitue le deuxième stade dudit procédé, est conduite en présence de nickel de Raney à une température de 120 à 140°C et sous une pression d'hydrogène d'au moins 42 $kg/cm^2$ pour obtenir de la méthyl-2 pentanediamine, le milieu réactionnel comprenant un solvant alcoolique qui renferme de l'ammoniac.

Toutefois la sélectivité en méthyl-2 pentanediamine n'est pas satisfaisante : elle reste comprise entre environ 55 et 60 %.

Pour résumer, aucun des procédés antérieurement connus ne donne pleinement satisfaction dans la mesure où il présente au moins l'un des inconvénients suivants :

– nécessité de travailler sous très haute pression pour obtenir un résultat appréciable avec un catalyseur à base de cobalt de Raney,
– manque de sélectivité en méthyl-2 pentanediamine soit au profit des produits plus lourds, soit au profit d'un autre produit non recherché ici (méthyl-3 pipéridine),
– manque d'efficacité des diverses techniques proposées notamment en raison des longues durées de finition requises.

La présente invention a donc pour objet un procédé de préparation de la méthyl-2 pentanediamine par hydrogénation en phase liquide de méthyl-2 glutaronitrile en présence d'un catalyseur à base de nickel de Raney, en milieu basique initialement non ammoniacal caractérisé en ce que la réaction d'hydrogénation est conduite à une température comprise entre 40 et 150°C, sous une pression totale inférieure à 40 bars, dans

un milieu réactionnel renfermant au maximum 10 % en poids d'eau, la concentration du méthyl-2 glutaronitrile dans le milieu réactionnel étant la plus faible possible.

Le procédé conforme à la présente invention est mis en oeuvre en phase liquide, en présence d'un catalyseur à base de nickel de Raney.

La réaction en cause se déroule entre le méthyl-2 glutaronitrile liquide, le cas échéant, introduit dans un solvant ou diluant et l'hydrogène gazeux sous pression en présence d'un catalyseur généralement solide. Il est donc important qu'elle soit conduite dans un appareillage dont la conception et le mode de fonctionnement favorisent le contact de ces 3 phases.

Par "catalyseur à base de nickel de Raney" (ou du type de Raney), on entend essentiellement des formes catalytiques particulières et de grande surface du nickel éventuellement dopé ou stabilisé par du chrome et par du fer, par exemple. Ces formes catalytiques particulières sont généralement obtenues, de manière en soi connue, à partir d'un alliage contenant du nickel, le cas échéant un ou plusieurs dopant(s), et de l'aluminium, le nickel étant présent à l'état "dissout" dans l'aluminium. Ces alliages sont alors soumis à un lessivage alcalin en vue d'éliminer la majeure partie de l'aluminium qu'ils renferment. Ces alliages peuvent néanmoins renfermer après traitement jusqu'à environ 20 à 30 % en poids d'aluminium.

Les références ci-après sont citées à titre représentatif des connaissances générales en la matière et notamment à propos des diverses préparations :

– Chemical Technology Review n° 94, "Hydrogenation catalysts" R. J. PETERSON/NOYES DATA Corp. 1977, "Preparation of Nickel Hydrogenation catalyst" pages 3-10.
– R. L. AUGUSTINE "Catalytic Hydrogenation", Marcel Dekker Inc. NY 1965, pages 26-32, et annexes pages 147-149.

De nombreux catalyseurs à base de Nickel de Raney sont des produits du commerce et conviennent à la mise en oeuvre du procédé selon l'invention.

La réaction d'hydrogénation est conduite dans un milieu basique initialement non ammoniacal.

La basicité initiale du milieu est assurée par la présence d'un hydroxyde de métal alcalin tel le sodium, le potassium ou le lithium, ou d'un hydroxyde d'ammonium quaternaire, à l'exclusion de l'ammoniac ou de solutions ammoniacales. Bien entendu, comme cela est bien connu dans l'art, il se forme au cours de l'hydrogénation en cause, de l'ammoniac qui est susceptible d'être transformé au moins pour partie en ammoniaque en présence d'eau. Toutefois dans le cadre de la présente invention, il est essentiel de ne pas conduire la réaction d'hydrogénation en présence d'une quantité supplémentaire d'ammoniac ou d'ammoniaque. C'est ce que la demanderesse entend par le fait que le milieu basique en cause est "initialement non ammoniacal".

Par hydroxyde d'ammonium quaternaire, en entend les composés hydroxylés dont le cation associé répond à l'une quelconque des formules (I) à (III) ci-après :

I)

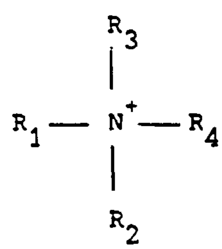

II)

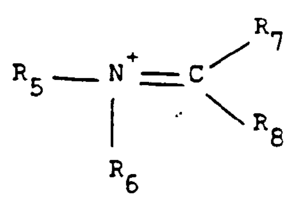

III) 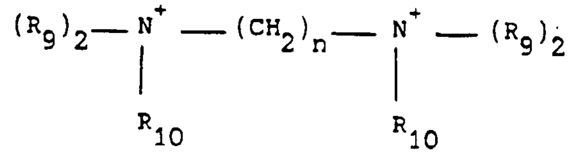

dans lesquelles :

- $R_1$, $R_2$, $R_3$, $R_4$ sont identiques ou différents et représentent :
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone ;
  . un radical aryle contenant de 6 à 10 atomes de carbone, éventuellement substitué par un ou des radicaux alkyles contenant de 1 à 4 atomes de carbone, alcoxy, alcoxycarbonyle ou halogéno ;
  . deux desdits radicaux $R_1$ à $R_4$ pouvant former ensemble un radical alkylène, alcénylène ou alcadiènylène, linéaire ou ramifié et contenant de 3 à 6 atomes de carbone ;
- $R_5$, $R_6$, $R_7$, $R_8$ sont identiques ou différents et représentent :
  . un radical alkyle linéaire ou ramifié contenant de 1 à 4 atomes de carbone ;
  . les radicaux $R_7$ et $R_8$ pouvant former ensemble un radical alkylène contenant de 3 à 6 atomes de carbone ;
  . les radicaux $R_8$ et $R_7$ ou $R_8$ et $R_8$ pouvant former ensemble un radical alkylène, alcénylène ou alcadiénylène, contenant 4 atomes de carbone et constituant avec N un hétérocycle azoté ;
- $R_9$ représente un radical alkyle linéraire ou ramifié contenant de 1 à 4 atomes de carbone ou un radical phényle ;
- $R_{10}$ représente :
  . un radical alkyle linéaire ou ramifié contenant de 1 à 4 atomes de carbone, semblable ou différent de $R_9$ ;
  . un radical alcényle linéaire ou ramifié, contenant de 2 à 12 atomes de carbone, de préférence de 4 à 8 atomes de carbone ;
- n représente un nombre entier supérieur ou égal à 1 et inférieur ou égal à 10 et de préférence inférieur ou égal à 6.

A titre d'exemple de cations onium quaternaire répondant à la formule I, on peut citer les cations :

. tétraméthylammonium,
. triéthylméthylammonium,
. tributylméthylammonium,
. triméthyl(n-propyl)ammonium,
. tétraéthylammonium,
. tétrabutylammonium,
. dodécyltriméthylammonium,
. méthyltrioctylammonium,
. heptyltributylammonium,
. tétrapropylammonium,
. tétrapentylammonium,
. tétrahexylammonium,
. tétraheptylammonium,
. tétraoctylammonium,
. tétradécylammonium,
. butyltripropylammonium,
. méthyltributylammonium,
. pentyltributylammonium,
. méthyldiéthylpropylammonium,
. éthyldiméthylpropylammonium,
. tétradodécylammonium,
. tétraoctadécylammonium,
. hexadécyltriméthylammonium,
. benzyltriméthylammonium,

. benzyldiméthylpropylammonium,
. benzyldiméthyloctylammonium,
. benzyltributylammonium,
. benzyltriéthylammonium,
. phényltriméthylammonium,
. benzyldiméthyltétradécylammonium,
. benzyldiméthylhexadécylammonium,
. diméthyldiphénylammonium,
. méthyltriphénylammonium,
. N,N-diméthyl-tétraméthylènammonium,
. N,N-diéthyl-tétraméthylènammonium.

Parmi les cations répondant à la formule II, on peut citer les cations :

. N-méthylpyridinium,
. N-éthylpyridinium,
. N-hexadécylpyridinium,
. N-méthylpicolinium.

Parmi les cations répondant à la formule III, on peut citer les cations :

. bis(triméthylammonium)-1,2 éthane,
. bis(triméthylammonium)-1,3 propane,
. bis(triméthylammonium)-1,4 butane,
. bis(triméthylammonium)-1,3 butane.

Parmi ces hydroxydes d'ammonium quaternaire, on préfère ceux dont le cation associé répond à la formule (I) ci-avant dans laquelle au moins trois quelconques des radicaux $R_1$ à $R_4$ sont identiques et choisis parmi les radicaux alkyles comportant de 1 à 10 atomes de carbone et, de préférence de 1 à 4 atomes de carbone, le dernier de ces radicaux étant choisi parmi les radicaux alkyles, aryles et aralkyles qui comportent au maximum 10 atomes de carbone.

A titre d'exemple de tels cations on ne peut citer les cations:

. tétraméthylammonium,
. tétraéthylammonium,
. benzyltriméthylammonium,
. phényltriméthylammonium.

Parmi les hydroxydes de métaux alcalins, on préfère l'hydroxyde de sodium et l'hydroxyde de potassium.

Le milieu réactionnel basique peut comprendre un diluant exogène et plus particulièrement un alcanol comportant de 1 à 12 atomes de carbone. De préférence, on utilise un alcanol comportant de 1 à 4 atomes de carbone et plus particulièrement, de l'éthanol ou de l'isopropanol.

Le milieu réactionnel peut ne renfermer aucun diluant exogène ; il contiendra alors un diluant endogène qui est de la méthyl-2 pentanediamine, le cas échéant, en mélange avec de la méthyl-3 pipéridine. L'agent basique (hydroxyde dont il a été question ci-avant) sera alors dissout ou dispersé au sein du diluant.

Les concentrations de l'agent basique et du catalyseur à base de nickel de Raney dans le milieu réactionnel peuvent varier dans de larges limites.

Le catalyseur (exprimé en nickel) peut représenter de 0,5 à 50 % en poids du diluant (endogène ou exogène) et, de préférence il représente de 1 à 15 % en poids du diluant.

L'hydroxyde de métal alcalin ou d'ammonium quaternaire peut représenter de 0,5 à 50 % (molaire) par rapport au nickel et, de préférence il représente de 2 à 35 % (molaire) par rapport au nickel.

La réaction d'hydrogénation est mise en oeuvre à une température comprise entre 40 et 150°C ; au-dessous de 40°C, la vitesse de réaction est insuffisante et au-dessus de 150°C, la formation de produits lourds devient rédhibitoire. Cette température est avantageusement comprise entre 60 et 110°C.

La pression totale mesurée à la température de réaction est inférieure à 40 bars. Elle est avantageusement supérieure à 5 bars et, de préférence, comprise entre 5 et 25 bars.

Il est essentiel à la mise en oeuvre du procédé selon l'invention de limiter la teneur en eau du milieu réactionnel de telle sorte que cell-ci soit au plus égale à 10 % en poids et, de préférence, inférieure ou égale à 4 % en poids dudit milieu.

Comme indiqué en tête du présent mémoire, il est essentiel à la mise en oeuvre du procédé selon l'invention que la concentration du méthyl-2 glutaronitrile dans le milieu réactionnel soit inférieure à 20% en poids dudit milieu.

Bien qu'en principe, il soit possible d'obtenir sélectivement le produit recherché en travaillant selon un mode opératoire par charges successives, ce mode discontinu nécessite de travailler avec une dilution importante du méthyl-2 glutaronitrile, c'est à dire une concentration initiale de cette matière première inférieure à 20

% en poids du milieu réactionnel et, de préférence, inférieure à 10 % en poids.

Selon un mode avantageux de réalisation de la présente invention, on opère selon un mode au moins semi-continu en ce sens que le méthyl-2 glutaronitrile, pris isolément ou en dilution dans un diluant dont la nature a déjà été précisée ci-avant, est introduit de telle sorte qu'à tout moment de la réaction, la consommation d'hydrogène soit de 4 moles par mole de méthyl-2 glutaronitrile engagée avec une tolérance de 3% en excès ou par défaut et que la concentration en méthyl-2 glutaronitrile du milieu réactionnel, suivie par analyse, soit inférieure 0,1 % en poids.

Pour une bonne mise en oeuvre de la présente invention la quantité totale de méthyl-2 glutaronitrile à hydrogéner sera au moins égale à 10 % en poids du diluant.

Bien entendu, le procédé peut être conduit en continu en prévoyant en outre l'alimentation continue d'hydrogène et le soutirage continu du milieu réactionnel, la décantation et/ou la filtration pour séparer le catalyseur du mélange liquide issu de la zone réactionnelle, la séparation du produit recherché par distillation et, le cas échéant, le recyclage d'au moins une partie du catalyseur et du diluant rendu basique accompagné d'une alimentation d'appoint en catalyseur, en diluant et en hydroxyde alcalin ou d'ammonium quaternaire.

Les exemples ci-après illustrent l'invention. Les conventions suivantes y sont utilisées :

TT : représente le taux de transformation du méthyl-2 glutaronitrile.

RR : représente le nombre de moles du produit considéré obtenu pour 100 moles de méthyl-2 glutaronitrile engagé.

mpda : méthyl-2 pentanediamine.

mpp : méthylpipéridine.

## EXEMPLES

Les quantités de nickel de Raney qui y sont mentionnées le sont pour la matière sèche.

### Exemple 1 :

Dans un autoclave en acier inoxydable de 3,6 litres de capacité équipé d'un agitateur à ancre tournant à 240 tours/minute et de trois contre-pales (la conception du réacteur et l'efficacité de l'agitation étant telles que compte tenu de la consommation d'hydrogène par unité de temps, la réaction ne soit jamais limitée par la transfert du gaz dans la phase liquide) on charge :

– 1 060 ml d'éthanol à 4 % (en poids) d'eau,

– 120 g de nickel de Raney (Ni 50) commercialisé par la société Procatalyse, préparé par attaque alcaline d'un alliage de nickel et d'aluminium comportant une proportion équipondérale de nickel et d'aluminium, l'alliage étant dopé au chrome),

– 31 g de soude en pastille.

L'autoclave purgé à l'azote est alors pressurisé à 15 bars d'hydrogène et chauffé à 100°C. On injecte alors en 4 heures 13,1 moles de méthyl-2 glutaronitrile (de pureté supérieure à 99,5 %). On poursuit la réaction pendant environ 1 heure 30 minutes après la fin de l'injection, en maintenant les conditions de température et de pression. Cette période appelée durée de finition est le temps nécessaire pour que la composition du milieu réactionnel suvie par chromatographie en phase gazeuse n'évolue plus.

En fin d'essai, le mélange réactionnel est refroidi puis filtré. Il est alors analysé par chromatographie en phase gazeuse.

Les résultats obtenus sont les suivants

| | |
|---|---|
| TT = | 100 % |
| RP (mpda) = | 86 % |
| RR (mpp) = | 10 % |
| RR (autres produits) = | 4 % |

### Exemples 2 à 4 ; essai témoin a :

Selon un mode opératoire analogue à celui décrit pour l'exemple 1, on réalise une série d'essais dans les conditions communes suivantes :

– on utilise un réacteur de 300 ml de capacité ;

– on injecte au total 0,1 mole de méthyl-2 glutaronitile ;

– on utilise un diluant exogène (100 ml) et du nickel de Raney (Ni 50, défini précédemment) à raison de 2,16 g ;

– la température est de 65°C ;

– la pression d'hydrogène est de 15 bars.

Les conditions particulières ainsi que les résultats obtenus sont rassemblés dans le tableau ci-après : TT = 100 % dans tous les essais.

| N° | Diluant | Agent basique | | Durée en mn | | RR(mpda) |
|---|---|---|---|---|---|---|
| | | Nature | (g) | Injection | Finition | % |
| 2 | ) Ethanol | KOH | 0,58 | 260 | 30 | 89 |
| 3 | ) | NaOH | 0,56 | 130 | 20 | 92 |
| a | ) absolu | $NH_4OH$ | 0,24 | 180 | 30 | 16 |
| 4 | Ethanol à 4% (pds) d'eau | NaOH | 0,56 | 180 | 30 | 86 |

Exemple 5 - Essai témoin b :

On reproduit l'exemple 4 ci-avant en modifiant la nature du diluant et la température de réaction qui est maintenant de 100°C.

Dans l'exemple 5, le diluant est de l'isopropanol anhydre ; dans l'essai témoin b, le diluant est de l'isopropanol renfermant 5 % en poids d'eau. La durée d'injection est de 90 mn, celle de la finition de 30 mn. Dans les deux cas TT = 100 %, mais les sélectivités sont très différentes :

RR (mpda) dans l'exemple 5 : 95,5 %

RR (mpda) dans l'essai b : 5 %.

Exemple 6 :

Dans un autoclave de 300 ml de capacité, on charge :

– 100 ml de méthyl-2 pentanediamine (diluant),
– 12,9 g de nickel de Raney (Ni 50 défini précédemment),
– 0,56 g de soude en pastilles.

L'autoclave purgé à l'azote est alors pressurisé à 15 bars d'hydrogène et chauffé a 100°C. On injecte alors en 3 heures 1,03 mole de méthyl-2 glutaronitrile puis on maintient les conditions de température et de pression pendant 30 minutes.

On obtient les résultats suivants :

TT = 100 % RR (mpda) = 88,1 %.

Exemple 7 :

Dans un appareillage analogue à celui décrit pour les exemples 2 à 4, on réalise un essai sur une charge renfermant :

– 0,1 mole de méthyl-2 glutaronitrile,
– 100 ml d'éthanol à 100 %,
– 2,16 g de nickel de Raney (Ni 50 décrit précédemment),
– 0,58 g de potasse en pastilles.

Après 7 heures de contact à 40°C sous 14 bars, on obtient les résultats suivants :

TT = 100 % RR (mpda) = 75,9 %.

Exemple 8 :

Dans un autoclave en acier inoxydable d'1 l de capacité, chauffé par une double enveloppe, agité par une turbine à 6 pales, muni de 4 contre-pales et purgé à l'azote, on charge :

– 300 ml d'éthanol à 96 %,
– 33 g de nickel Raney (NI 50 précédemment défini),
– 1,7 g d'une solution aqueuse de soude à 50 %.

On pressurise le réacteur avec de l'hydrogène, on met l'agitation en route à 1 500 tr/mn et on monte la température à 50°C environ.

On règle alors la pression à 5 bars dans le réacteur puis on introduit 2,82 moles de méthyl-2 glutaronitrile en 3 h 40 mn, la température étant contrôlée pour être maintenue à 65°C.

On stoppe alors l'introduction de méthyl-2 glutaronitrile et on maintient les conditions de température et de pression pendant 30 mn.

TT = 100 %

RR (mpda) = 72,7 %, ce qui correspond à une productivité horaire de 0,56 mole de méthyl-2 pentanediamine.

Exemple 9 :

On reproduit l'exemple 8 ci-avant en modifiant la pression d'hydrogène ; la pression dans le réacteur est maintenant maintenue à 10 bars.

On alimente 3,52 moles de méthyl-2 glutaronitrile en 4 h 40 mn et on maintient les conditions de température et de pression pendant 30 mn après la fin de l'injection. Les résultats sont les suivants :

TT = 100 %  RR (mpda) = 79,7 %,

ce qui correspond à une productivité horaire de 0,6 mole de méthyl-2 pentanediamine.

## Revendications

1. Procédé de fabrication de la méthyl-2 pentanediamine par hydrogénation en phase liquide du méthyl-2 glutaronitrile, en présence d'un catalyseur à base de nickel de Raney, en milieu basique initialement non-ammoniacal, caractérisé en ce que la réaction d'hydrogénation est conduite à une température comprise entre 40 et 150°C, sous une pression totale inférieure à 40 bars, dans un milieu réactionnel renfermant au maximum 10 % en poids d'eau, la concentration initiale du méthyl-2 glutaronitrile étant inférieure à 20% en poids du milieu réactionnel.

2. Procédé selon la revendication 1, caractérisé en ce que la température de réaction est comprise entre 60 et 110°C.

3. Procédé selon la revendication 1 ou 2, caractérisé en ce que la pression d'hydrogène est inférieure à 25 bars.

4. Procédé selon l'une quelconque des revendications précédentes, caractérisé en ce que le milieu réactionnel renferme au maximum 4 % en poids d'eau.

5. Procédé selon l'une quelconque des revendications précédentes, caractérisé en ce que le milieu réactionnel renferme un alcanol comportant de 1 à 12 atomes de carbone.

6. Procédé selon la revendication 5, caractérisé en ce que l'alcanol est choisi parmi l'éthanol et l'isopropanol.

7. Procédé selon l'une quelconque des revendications 1 à 4, caractérisé en ce que le milieu réactionnel est exempt de diluant exogène au système réactionnel et renferme comme diluant de la méthyl-2 pentanediamine, le cas échéant, en mélange avec de la méthyl-3 pipéridine.

8. Procédé selon l'une quelconque des revendications précédentes, caractérisé en ce que le milieu réactionnel renferme de l'hydroxyde de sodium ou de l'hydroxyde de potassium

9. Procédé selon l'une quelconque des revendications précédentes, caractérisé en ce que le catalyseur (exprimé en nickel) représente de 0,5 à 50 % et, de préférence, de 1 à 15 % en poids du diluant.

10. Procédé selon la revendication 9, caractérisé en ce que l'hydroxyde alcalin ou d'ammonium quaternaire représente de 0,5 à 50 % et, de préférence, de 2 à 35 % (molaire) par rapport au nickel.

11. Procédé selon l'une quelconque des revendications précédentes, caractérisé en ce que la concentration initiale du méthyl-2 glutaronitrile est inférieure à 10 % en poids du milieu réactionnel.

12. Procédé selon l'une quelconque des revendications précédentes, caractérisé en ce que le méthyl-2 glutaronitrile est introduit dans le milieu réactionnel de telle sorte qu'à tout moment de la réaction, la consommation d'hydrogène soit de 4 moles par mole de méthyl-2 glutaronitrile engagée avec une tolérance de 3 % en excès ou par défaut et que la concentration en méthyl-2 glutaronitrile du milieu réactionnel, suivie par analyse, soit inférieure à 0,1 % en poids.

## Patentansprüche

1. Verfahren zur Herstellung von 2-Methyl-pentandiamin durch Hydrieren von 2-Methyl-glutarnitril in flüssiger Phase in Gegenwart eines Katalysators auf der Basis von Raney-Nickel, in einem ursprünglich nicht-ammoniakalischen basischen Medium, dadurch gekennzeichnet, daß die Hydrierungsreaktion bei einer Temperatur im Bereich von 40 bis 150°C unter einem Gesamtdruck von weniger als 40 bar in einem Reaktionsmedium ausgeführt wird, das maximal 10 Gew.-% Wasser enthält, wobei die Anfangskonzentration des 2-Methyl-glutarnitrils weniger als 20 Gew.-% des Reaktionsmediums ausmacht.

2. Verfahren nach Anspruch 1, dadurch gekennzeichnet, daß die Reaktionstemperatur 60 bis 110°C beträgt.

3. Verfahren nach Anspruch 1 oder 2, dadurch gekennzeichnet, daß der Wasserstoffdruck weniger als 25 bar beträgt.

4. Verfahren nach einem der vorangehenden Ansprüche, dadurch gekennzeichnet, daß das Reaktionsmedium maximal 4 Gew.-% Wasser enthält.

5. Verfahren nach einem der vorangehenden Ansprüche, dadurch gekennzeichnet, daß das Reaktionsmedium ein Alkanol mit 1 bis 12 kohlenstoffatomer. enthält.

6. Verfahren nach Anspruch 5, dadurch gekennzeichnet, daß das Alkanol aus Ethanol und Isopropanol ausgewählt wird.

7. Verfahren nach einem der Ansprüche 1 bis 4, dadurch gekennzeichnet, daß das Reaktionsmedium frei ist von einem für das Reaktionssystem exogenen Verdünnungsmittel und als Verdünnungsmittel 2-Methyl-pentandiamin, gegebenenfalls im Gemisch mit 3-Methyl-piperidin enthält.

8. Verfahren nach einem der vorangehenden Ansprüche, dadurch gekennzeichnet, daß das Reaktionsmedium Natriumhydroxid oder Kaliumhvdroxid enthält.

9. Verfahren nach einem der vorangehenden Ansprüche, dadurch gekennzeichnet, daß der Katalysator (angegeben als Nickel) 0,5 bis 50 Gew.-%, vorzugsweise 1 bis 15 Gew.-%, des Verdünnungsmittels ausmacht.

10. Verfahren nach Anspruch 9, dadurch gekennzeichnet, daß das Alkali- oder quaternäre Ammoniumhydroxid 0,5 bis 50 mol%, vorzugsweise 2 bis 35 mol%, bezogen auf Nickel, ausmacht.

11. Verfahren nach einem der vorangehenden Ansprüche, dadurch gekennzeichnet, daß die Anfangskonzentration an 2-Methyl-glutarnitril weniger als 10 Gew.-% des Reaktionsmediums ausmacht.

12. Verfahren nach einem der vorangehenden Ansprüche, dadurch gekennzeichnet, daß das 2-Methyl-glutarnitril in das Reaktionsmedium so eingebracht wird, daß zu jedem Zeitpunkt der Reaktion der Wasserstoffverbrauch 4 mol je mol eingesetztes 2-Methyl-glutarnitril beträgt mit einer Toleranz von 3% Überschuß oder Unterschuß und daß die mittels Analyse kontrollierte Konzentration des Reaktionsmediums an 2-Methyl-glutarnitril weniger als 0,1 Gew.-% ausmacht.

## Claims

1. Process for the manufacture of 2-methylpentanediamine by liquid-phase hydrogenation of 2-methylglutatonitrile in the presence of a Raney nickel-based catalyst, in an initially nonammoniacal basic medium, characterised in that the hydrogenation reaction,is performed at a temperature of between 40 and 150°C, at a total pressure below 40 bars, in a reaction medium containing not more than 10% by weight of water, the initial concenttation of 2-methylglutaronitrile being below 20% by weight of the reaction medium.

2. Process according to Claim 1, characterised in that the reaction temperature is between 60 and 110°C.

3. Process according to Claim 1 or 2, characterised in that the hydrogen pressure is below 25 bars

4. Process according to any one of the preceding claims, characterised in that the reaction medium includes not more than 4% by weight if water.

5. Process according to any one of the preceding claims, characterised in that the reaction medium includes an alkanol containing from 1 to 12 carbon atoms.

6. Process according to Claim 5, characterised in that the alkanol is chosen from ethanol and isopropanol.

7. Process according to any one of Claims 1 to 4, characterised in that the reaction medium is free from any diluent exogenous to the reaction system and includes 2-methylpentanediamine as diluent mixed, if appropriate, with 3-methylpiperidine.

8. Process according to any one of the preceding claims, characterised in that the reaction medium includes sodium hydroxide or potassium hydroxide.

9. Process according to any one of the preceding claims, characterised in that the catalyst (expressed as nickel) represents from 0.5 to 50% and, preferably, from 1 to 15% by weight of the diluent.

10. Process according to Claim 9, characterised in that the alkali metal or quaternary ammonium hydroxide

represents from 0.5 to 50% and, preferably, from 2 to 35% (molar) in relation to nickel.

11. Process according to any of the preceding claims, characterised in that the initial concentration of 2-methylglutaronitrile is below 10% by weight of the reaction medium.

12. Process according to any one of the preceding claims, characterised in that 2-methylglutaronitrile is introduced into the reaction medium so that at any time during the reaction the hydrogen consumption is 4 moles per mole of 2-methylglutaronitrile employed, with a tolerance of 3% in excess or in deficiency, and that the concentration of 2-methylglutaronitrile in the reaction medium, monitored by analysis, is below 0.1% by weight.